# EUROPEAN PATENT APPLICATION

(11) **EP 4 382 119 A1**
(43) Date of publication of application: **12.06.2024**
(21) Application number: 22212572.6
(22) Date of filing: 09.12.2022
(51) Int. Cl.: A61K 39/00

(54) **A KIT FOR USE IN THE TREATMENT OF HEMATOLOGICAL CANCER**

(71) Applicant: AvenCell Europe GmbH, 01307 Dresden (DE)
(72) Inventor: Ehninger, Gerhard, 01307 Dresden (DE); Cartellieri, Marc, 01307 Dresden (DE); Ehninger, Armin, 01307 Dresden (DE)
(74) Representative: Kailuweit & Uhlemann Patentanwälte Partnerschaft mbB

(57) **Abstract**

The present invention relates to a kit for use in the treatment of hematological cancer comprising a T cell comprising a nucleotide sequence encoding a switchable chimeric antigen receptor (CAR) and a targeting module comprising at least one hematological cancer cell-binding domain and a tag-binding domain or a tag, wherein at least one dosage of a clinically effective amount of the T cell is administered to a subject having hematological cancer and wherein a first dosage of the targeting module is continuously administered to the subject.

## Description

The present invention relates to a kit for use in the treatment of hematological cancer comprising a T cell comprising a nucleotide sequence encoding a switchable chimeric antigen receptor (CAR) and a targeting module comprising at least one hematological cancer cell-binding domain and a tag-binding domain or a tag, wherein at least one dosage of a clinically effective amount of the T cell is administered to a subject having hematological cancer and wherein a first dosage of the targeting module is continuously administered to the subject.

Chimeric antigen receptors (CARs) are artificial receptors consisting of a binding moiety, which provides the antigen-specificity, e.g. a single chain variable fragment (scFv) recognizing a surface antigen of a tumor cell, a transmembrane domain, and one or several signaling chains derived from immune receptors, e.g. CD3ζ, that activates an immune cell (Cartellieri *et al.* 2010).

Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al.* 2010). Moreover, as CAR-T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CAR T cells to very few indications. In fact, examples of clinical effectiveness have been restricted to CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri *et al.* 2016). Antigen-specificity is provided by soluble adapter proteins, which consist of an antigen-binding domain fused to the tag recognized by the universal CAR. Cartellieri *et al.* describe the treatment of CD33- and/or CD123-positive acute myeloid leukemia cells *in vitro* and *in vivo.*

Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell-binding domain (EP 3 581 200 A1).

Moreover, switchable CAR-T approaches like UniCAR or RevCAR provide the possibility to rest CAR T cells in-between cycles of activation and stimulation by pausing administration of the soluble adapter molecule. This is expected to prevent the exhaustion observed upon continuous stimulation of conventional CAR T cells thereby improving persistence (Weber *et al*. 2021).

WO 2018/160622 A1 discloses methods of treating a patient with a cancer by administering to the patient a composition comprising CAR T cells and administering to the patient a small molecule linked to a targeting moiety by a linker, to reduce off-target toxicity and more precisely controlling CAR T cell activation. The small molecule ligand linked to a targeting moiety by a linker is used as a bridge between the cancer and the CAR T cells directing the CAR T cells to the cancer for amelioration of the cancer. WO 2018/160622 A1 describes a method of treatment of a cancer, wherein the small molecule is administered before the CAR T cells, in particular a first and second dose of CAR T cells; a method, wherein the small molecule is administered before the CAR T cells which are administered as mixture of CAR T cells and non-transformed T cells; a method furthermore comprising the administration of folate; and a method, wherein the small molecule is administered continuously, then CAR T cells are administered and the continuously administration of the targeting module is ended to inhibit or prevent cytokine release syndrome in the patient.

The object of the present invention is to provide a kit for use in the treatment of hematological cancer with reduced side effects and/or improved efficacy.

The object has been solved by a first aspect of the invention is a kit for use in the treatment of hematological cancer comprising:
a. a T cell comprising a nucleotide sequence encoding a switchable chimeric antigen receptor (CAR), wherein the switchable CAR comprises
   a tag-binding domain or tag,
   an extracellular hinge and a transmembrane domain, and
   an intracellular signaling domain that comprises at least one signal transduction domain; and
b. a targeting module comprising
   at least one hematological cancer cell-binding domain and
   a tag-binding domain or a tag,

wherein the tag-binding domain of the targeting module binds to the tag of the switchable CAR or the tag of the targeting module binds to the tag-binding domain of the switchable CAR,
wherein a first dosage of the targeting module is continuously administered to a subject having hematological cancer with a dosage quantity in the range of 0.1 mg/day to 20 mg/day from day 0 of the treatment (beginning of the treatment) for a period between 10 days and 25 days, and
wherein at least one dosage of a clinically effective amount of the T cell comprising a nucleotide sequence encoding a switchable CAR is administered to the subject with a dosage quantity in the range of 1·10⁸ to 1·10⁹ cells once within day 0 to day 5 of the treatment, and
wherein 7 days to 21 days after the administering of the first dosage of the targeting module at least one further dosage of the targeting module is continuously administered to the subject with a dosage quantity in the range of 0.1 mg/day to 20 mg/day for a period between 4 days and 25 days.

According to the invention, the first dosage of the targeting module is administered to the subject prior to, concurrently with and after the T cell comprising a nucleotide sequence encoding a switchable CAR. Advantageously, the administration prior to the administration of the T cell comprising a nucleotide sequence encoding a switchable CAR enables the stimulation of the switchable CAR upon infusion, which is critical for engraftment and expansion of the T cells.

According to the invention, the kit is used for a combination therapy and the treatment is initiated on day 0 of the combination therapy.

Advantageously, the switchable CAR according to the invention used in combination with a targeting module actively targets target cells, e.g. tumor cells, and is capable of inducing a significant anti-tumor response, wherein the anti-tumor response of the switchable chimeric antigen receptor is only induced in the presence of the targeting module. The effect can be reversibly interrupted by withholding the administration of the targeting module. Further advantageously, the pharmacokinetic and pharmacodynamic half-life of the targeting module is short, providing a rapid and reversible switch-off mechanism of the mediated immune response. Advantageously, using the dosage regime according to the invention side effects, such as fever (≥ 38°C), could effectively be reduced.

According to the invention, the first dosage of the targeting module serves as induction cycle for the therapy and the at least one further dosage of the targeting module serves as consolidation cycle. Preferably, the first dosage and/or at least one further dosage are continuously administered to the subject to achieve a steady-state concentration of targeting module in the subject.

As used herein, the term "continuously" refers to an administration with nearly no interruption or gap, preferably without any interruption and gaps. The term comprises the administration with interruptions for 1 min to 72 h, because of a change of an infusion or subsiding of side effects.

As used herein, the term "switchable chimeric antigen receptor" refers to an artificial chimeric fusion protein, in particular a receptor comprising a tag or a tag-binding domain, an extracellular hinge and a transmembrane domain and an intracellular signaling domain. The domains can be derived from different sources and therefore, the receptor is called chimeric. Advantageously, the receptor can bind with the tag or tag-binding domain to the tag-binding domain or tag of different targeting modules, which in turn bind to an antigen on a target cell, in particular the hematological cancer cell. Thus, the tag or tag-binding domain serves as target cell-binding domain. According to the invention, the T cell comprising a nucleotide sequence encoding a switchable CAR expresses the switchable CAR comprising a tag-binding domain or tag, an extracellular hinge and a transmembrane domain, and an intracellular signaling domain that comprises at least one signal transduction domain.

As used herein, the term "domain" refers to a part of a protein sequence, which can exist and function independently from the rest of the protein.

In embodiments, the targeting module and/or the T cell comprising a nucleotide sequence encoding a switchable CAR are administered in form of a pharmaceutical composition.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1 ng/ml to 500 mg/ml of the targeting module, preferably in the range of 1 µg/ml to 5 mg/ml.

In embodiments, the pharmaceutical composition is an injectable buffered solution comprising a concentration in the range of 1•10⁵ to 1•10^{$} per mL of the T cell comprising a nucleotide sequence encoding the switchable CAR, and optionally, a concentration in the range of 1 ng/ml to 500 mg/ml of the targeting module.

In embodiments, the pharmaceutical composition comprises the targeting module and/or the T cell comprising a nucleotide sequence encoding a switchable CAR and a pharmaceutically acceptable thinner (dilution agent) or carrier.

In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In further embodiments, the pharmaceutical composition is sterile. The pharmaceutical composition is sterilized by conventional well-known techniques including, but not limited to, sterile filtration.

In embodiments, the pharmaceutical composition is used for administration to the subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted h an infusion and stabilizer solution prior to administration to a subject. The solutions used for reconstitution or infusion/stabilization may contain any of the components mentioned for the pharmaceutical composition or similar components.

In embodiments, the hematological cancer is leukemia. In embodiments, the subject has acute myeloid leukemia (AML).

In embodiments, the subject is a mammal. In embodiments, the mammal is a human.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g. FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 18, SEQ ID No. 19 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 20 or SEQ ID No. 21 or mutants thereof; or a short linear peptide sequence from a human protein, preferably from a human nuclear protein, more preferably from the human La protein, even more preferably according to SEQ ID No. 22, SEQ ID No. 23, SEQ ID No. 24 or mutants thereof.

As used herein, the term "mutation" is intended the substitution, deletion, insertion of one or more amino acids in a polypeptide sequence.

As used herein, the term "nuclear protein" refers to a protein found in the cell nucleus. Advantageously, tags, which are peptide sequences from nuclear antigens, cannot be accessed and bound by the corresponding tag-binding domain naturally or under physiological conditions, respectively. This means that the tag-binding domain cannot bind the antigen naturally occurring in the cell nucleus and is thus not unintendedly bound in the absence of any administered tag or tag-comprising molecule. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the human protein is a human Alpha-fetoprotein and human nuclear proteins, more preferably from the human La protein.

In embodiments, the His-tag is an amino acid sequence consisting of histidine residues, preferably in the range of six to fourteen histidine residues.

In embodiments, the tag is a myc-tag, a His-tag, a short linear peptide sequence from yeast transcription factor GCN4, a leucine zipper sequence or a short linear peptide sequence from a human protein.

Preferably, the tag is the human La epitope E5B9 according to SEQ ID No. 22 or E7B6 according to SEQ ID No. 23 or SEQ ID No. 24, most preferably the human La epitope E5B9 according to SEQ ID No. 22 or E7B6 according to SEQ ID No. 24.

In embodiments, the tag is present at the amino-terminal end of the polypeptide that comprises the switchable CAR. Advantageously, locating the tag at the amino terminus permits the tag unhampered access to the targeting module that is bound to the target cell.

In embodiments, the tag-binding domain is an antibody, antigen-binding fragment, a protein or a peptide binding to a myc-tag, a His-tag, a short linear peptide sequence from yeast transcription factor GCN4, a leucine zipper sequence or a short linear peptide sequence from a human protein, preferably form a human nuclear protein, most preferably from the human La protein.

As used herein, the terms "peptide", "polypeptide" and "protein" are used interchangeably, and refer to a compound comprised of amino acid residues covalently linked by peptide bonds. A protein or peptide must contain at least three amino acids, and no limitation is placed on the maximum number of amino acids that can comprise a protein's or peptide's sequence.

Amino acid residues in a polypeptide sequence are designated herein according to the one-letter code, in which, for example, Q means Gln or Glutamine residue, R means Arg or Arginine residue and D means Asp or Aspartic acid residue.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment or antigen-binding fragment" refers to a protein comprising at least the V_{L} or V_{H} of an antibody. In embodiments, antibody fragments are selected from single-chain variable fragments (scFv), single-chain antibodies, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, V_{L} and V_{H} are connected via a glycine-serine linker with the structure (GₓS_{y}) with x and y selected from 1 to 10, preferably 3 to 5. Mostly preferred are 1 to 10 repeats of the sequence G₄S₁ (SEQ ID No. 1). Moreover, linkers are preferred that are constituted of a peptide sequence that can increase the protease resistance of the antibody derivatives.

In embodiments of the invention, the linker of the tag-binding domain comprises 20 to 30 amino acids, preferably 25 amino acids.

In further embodiments, the linker of the tag-binding domain comprises a linker according SEQ ID No. 2 or SEQ ID No. 3.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on in silico peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including in silico design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In preferred embodiments, the tag-binding domain is an antibody or an antibody fragment.

In embodiments, the tag-binding domain binds to a tag, wherein the tag is a short linear peptide sequence from the human La protein, preferably from the human La epitope E5B9 according to SEQ ID No. 22 or E7B6 according to SEQ ID No. 24.

In embodiments, the tag-binding domain is an antibody or an antigen-binding fragment, preferably comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 7), wherein X₂₄, X₃₅, X₆₁ and X₁₀₁ are independently from each other selected from a proteinogenic alpha-amino acid residue; or a sequence having at least 90 % sequence identity, preferably at least 95 % sequence identity; to sequence SEQ ID No. 8.

In embodiments, the tag-binding domain constitutes an anti-La epitope scFv.

In some embodiments, X₂₄, X₃₅, X₆₁ and X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

Preferably, the tag-binding domain comprises a sequence having each at least 90 % sequence identity, preferably at least 95 % sequence identity; to the sequences according to SEQ ID NO. 8 (V_{L}) and SEQ ID NO. 9 (V_{H}).

Most preferably, the tag-binding domain constitutes the anti-La 5B9 scFv according to SEQ ID NO. 8 (V_{L}) and SEQ ID NO. 9 (V_{H}).

In embodiments, the tag-binding domain is an antibody or antigen-binding fragment binding to the human La epitope E5B9 or E7B6 comprising a V_{L}-linker-V_{H} structure, wherein the V_{L} region of the tag-binding domain comprises a sequence with at least 95 % identity, preferably 99 % identity, with the sequence according to SEQ ID No. 8 or SEQ ID No. 25 and/or the V_{H} region of the tag-binding domain comprises a sequence with at least 95 % identity, preferably 99 % identity, with the sequence according to SEQ ID No. 9 or SEQ ID No. 26.

In embodiments, the at least one dosage of a clinically effective amount of the T cell comprising a nucleotide sequence encoding a switchable CAR is administered to the subject with a dosage quantity in the range of 1·10⁸ to 1·10⁹ cells once within day 0 to day 5 of the treatment.

In embodiments, two dosages of a clinically effective amount of the T cell comprising a nucleotide sequence encoding a switchable CAR are administered to the subject with a total dosage quantity of both dosages in the range of 1·10⁸ to 1·10⁹ cells within day 0 to day 5 of the treatment.

In embodiments, the at least one dosage of a clinically effective amount of the T cell comprising a nucleotide sequence encoding a switchable CAR is administrated with a dosage quantity in the range of 1·10⁸ to 5·10⁸ cells.

In embodiments, one dosage of a clinically effective amount of a T cell comprising a nucleotide sequence encoding a switchable CAR is administrated to the subject.

In embodiments, one dosage of a clinically effective amount of the T cell comprising a nucleotide sequence encoding a switchable CAR is administrated once on day 1 of the treatment.

In embodiments, one dosage of a clinically effective amount of the T cell comprising a nucleotide sequence encoding a switchable CAR is administrated 1 hour to 24 hours after starting the continuous administering of the targeting module.

In embodiments, at least one further dosage of a clinically effective amount of the T cell comprising a nucleotide sequence encoding a switchable CAR is administered to the subject with a dosage quantity in the range of 1·10⁸ to 1·10⁹ cells.

In embodiments, one further dosage of a clinically effective amount of the T cell comprising a nucleotide sequence encoding a switchable CAR is administered to the subject with a dosage quantity in the range of 1·10⁸ to 1·10⁹ cells.

In embodiments, two further dosages of a clinically effective amount of the T cell comprising a nucleotide sequence encoding a switchable CAR are administered to the subject with a dosage quantity in the range of 1·10⁸ to 1·10⁹ cells.

In embodiments, three further dosages of a clinically effective amount of the T cell comprising a nucleotide sequence encoding a switchable CAR are administered to the subject with a dosage quantity in the range of 1·10⁸ to 1·10⁹ cells.

As used herein, the term "targeting module" refers to a molecule, preferably a polypeptide or protein with at least two different domains, wherein each domain is specific for a target or a uniform group of targets, respectively, wherein at least one domain is specific for a target cell, in particular the hematological cancer cell-binding domain, e.g. a CD123-binding domain; and one domain is specific for a switchable chimeric antigen receptor, in particular the tag or tag-binding domain. In embodiments, the targeting module is isolated. As used herein, the term "isolated" means altered or removed from the natural state.

Preferably, the targeting module according to the invention is expressed as a recombinant protein. In further embodiments, the targeting module is chemically synthesized.

As used herein, the term "target cell-binding domain" refers to a peptide, protein, or low molecular weight organic ligand, which specifically binds a protein or protein complex (antigen) on the surface of a target cell, in particular the hematological cancer cell-binding domain.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In further embodiments, the hematological cancer cell-binding domain is a soluble T cell receptor consisting of the alpha and beta or the gamma and delta chain of a T cell receptor (TCR).

In embodiments, the targeting module is in monomeric, dimeric or polymeric form, preferably in monomeric form.

In further embodiments, the targeting module is monovalent, bivalent or multivalent.

In embodiments, the hematological cancer cell-binding domain is an antibody, an antibody fragment, a protein, a peptide or a low molecular weight organic ligand that binds to surface antigens selected from the group comprising CD19, CD20, CD22, CD30, CD33, CD38, CD56, CD70, CD123, CD133, CD371, CXCR4, members of the mucin protein family and ligands of the NKG2D receptor.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, in particular the hematological cancer cell.

In embodiments, the hematological cancer cell-binding domain is an antibody, an antibody fragment, a protein, a peptide or a low molecular weight organic ligand that binds toCD123.

In preferred embodiments, the CD123-binding domain is an antibody or antigen binding fragment.

In embodiments, the CD123-binding domain comprises the sequences SEQ ID No. 4 and SEQ ID No. 5.

In preferred embodiments, the CD123-binding domain comprises a sequence according to SEQ ID No. 6.

In embodiments, the hematological cancer cell-binding domain comprises a humanized amino acid sequence.

In embodiments, the different domains of the targeting module are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 1). Moreover, linkers are preferred that are constituted of a peptide sequence that can increase the protease resistance of the antibody derivatives.

In embodiments, the linker is SEQ ID No. 2 or SEQ ID No. 3.

In embodiments, the targeting module has a length in the range of 20 to 1600 amino acids, preferably 200 to 800 amino acids.

In embodiments, the targeting module comprises one of the sequences according to SEQ ID No. 10 to SEQ ID No. 17.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90% sequence identity to the half-life increasing domain, preferably at least 95% sequence identity. Advantageously, the mutant is capable of having one or more activities of the named peptides or proteins; in particular, the mutant increases the half-life like the half-life increasing domain.

In embodiments, the first dosage and/or the at least one further dosage of the targeting module is administered in the range of 0.5 mg/day to 8 mg/day, preferably in the range of 0.5 mg/day to 2 mg/day.

In embodiments, the first dosage of the targeting module is continuously administered for a period between 20 days and 24 days, preferably for a period of 20 days or 21 days.

As used herein, the term "extracellular hinge and transmembrane domain" refers to a flexible peptide sequence connected to the tag-binding domain or tag, which anchors the switchable CAR into the cell membrane of the cell and protrudes from the surface of the cell for optimal binding to its particular targeting module.

In embodiments, the extracellular hinge and transmembrane domain are selected from extracellular hinge and transmembrane domains of human CD8α, CD28, ICOS (CD278), NK cell receptors, preferably natural killer group NKG2D; or parts of the constant region of an antibody and combinations thereof. As used herein, the term "combinations thereof" refers to combinations of the different hinge and transmembrane domains.

In preferred embodiments, the extracellular hinge and transmembrane domain comprise an extracellular hinge and a transmembrane domain of CD8α, CD28, ICOS (CD278) or mutants and combinations thereof.

As used herein, the term "mutants" refers to peptides or proteins having at least 90% sequence identity to the named domains, antibodies, antibody fragments, peptides or proteins, preferably at least 95 % sequence identity. Advantageously, the mutants are capable of having one or more activities of the named domains, antibodies, antibody fragments, peptides or proteins.

In embodiments, the mutant comprises a point mutation. As used herein, a "point mutation" is a mutation, wherein a single nucleotide base is changed, inserted or deleted from nucleotide sequence.

In embodiments, mutants are truncated variants of peptides or proteins. As used herein, the term "truncated versions" refers to shortened peptides or proteins having at least 90% sequence identity to the named peptides or proteins, preferably at least 95 % sequence identity, more preferably having a chain length of at least 90 % and a sequence identity of 100 %, most preferably a chain length of at least 95 % and a sequence identity of 100 %, resulting from a mutation in the nucleotide sequence coding for the peptide or protein. Advantageously, the truncated version has at least 80%, preferably of at least 90 %, more preferably of at least 95 %; of the activity of the named peptide or protein.

Milone *et al.* and Zhao *et al.* describe the use of hinge and transmembrane domains of human CD8α molecule in CARs (Milone *et al.* 2009, Zhao *et al.* 2009).

Pinthus *et al.* and Cartellieri *et al.* describe the use of hinge and transmembrane domains of the human CD28 molecule in CARs (Pinthus *et al.* 2003, Cartellieri *et al.* 2016).

Zhang *et al.* describe the use of hinge and transmembrane domains of NKG2D in CARs (Zhang *et al.* 2005).

Frigault *et al.* and Wang *et al.* describe the use of hinge and transmembrane domains of parts of the constant region of immunoglobulin G1 (IgG) (Frigault *et al.* 2015, Wang *et al.* 2007). Frigault *et al.* describes the use of hinge domains of the constant region of IgG4.

In embodiments, combinations of the extracellular hinge and transmembrane domain are CD8α extracellular hinge and transmembrane domain, CD28 extracellular hinge and transmembrane domain, CD28 extracellular hinge domain combined with a CD8α or ICOS transmembrane domain, preferably a CD28 extracellular hinge and transmembrane domain or a CD28 extracellular hinge domain combined with an ICOS transmembrane domain.

In preferred embodiments, the extracellular hinge and transmembrane domain comprises an extracellular hinge domain of CD28, preferably a mutant of an extracellular hinge domain of CD28, more preferably a mutant of an extracellular hinge domain of CD28 comprising two point mutations in the B7 binding site. Advantageously, the mutation of the B7 binding site results in an abrogated ligand binding. Preferably, the extracellular hinge and transmembrane domain comprises a mutant of extracellular hinge domain of CD28 according to SEQ ID No. 27.

In embodiments, the extracellular hinge and transmembrane domain comprises SEQ ID No. 27 and SEQ ID No. 28 or SEQ ID No. 29.

As used herein, the term "signal transduction domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module.

In embodiments, the signal transduction domain is selected from cytoplasmic regions of CD3, CD28, CD137 (4-1BB), CD134 (OX40), CD278 (ICOS), DAP10, CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors, IL-2, IL-7, IL-15 or IL-21 and mutants thereof.

In preferred embodiments, the intracellular signaling domain comprises at least one signal transduction domain selected from the group consisting of a cytoplasmic region of CD3, CD28, 4-1BB (CD137), ICOS (CD278), IL-2, IL-7, IL-15 or IL-21 and mutants thereof.

In embodiments, the intracellular signaling domain comprises two or three signal transduction domains selected from the group comprising CD28, 4-1BB, ICOS, CD3ζ, IL-7Rα and mutants thereof.

In embodiments, the intracellular signaling domain comprises two or three signal transduction domains selected from the group comprising the sequences according to SEQ ID No. 30 to SEQ ID No. 38.

In embodiments, the intracellular signaling domain comprises at least a cytoplasmic region of CD28 or mutants thereof, preferably according to SEQ ID No. 30 or SEQ ID No. 31.

In embodiments, the intracellular signaling domain comprises at least a cytoplasmic region of CD3ζ or mutants thereof, preferably according to one of the sequences SEQ ID No. 32 to SEQ ID No. 34.

In embodiments, the intracellular signaling domain comprises at least a cytoplasmic region of CD28 or mutants thereof, preferably according to SEQ ID No. 30 or SEQ ID No. 31, and a cytoplasmic region of CD3ζ or mutants thereof, preferably according to one of the sequences SEQ ID No. 32 to SEQ ID No. 34.

In further embodiments, the intracellular signaling domain comprises at least a cytoplasmic region of ICOS or mutants thereof, preferably according to SEQ ID No. 35.

In embodiments, the intracellular signaling domain comprises at least a cytoplasmic region of ICOS or mutants thereof, preferably according to SEQ ID No. 35, and a cytoplasmic region of CD3ζ or mutants thereof, preferably according to one of the sequences SEQ ID No. 32 to SEQ ID No. 34.

Preferably, the switchable chimeric antigen receptor comprises a CD28 transmembrane domain and as intracellular signaling domain at least one cytoplasmic region of CD28 or mutants thereof, preferably according to SEQ ID No. 30 or SEQ ID No. 31, or an ICOS transmembrane domain and as intracellular signaling domain at least one cytoplasmic region of ICOS or mutants thereof, preferably according to SEQ ID No. 35.

In embodiments, the intracellular signaling domain comprises further a cytoplasmic region of a cytoplasmic region of 4-1BB, preferably according to SEQ ID No. 36, IL-2Rβ, IL-7Rα and mutants thereof, preferably according to SEQ ID No. 37 or 38, more preferably a cytoplasmic region of 4-1BB, IL-7Rα and mutants thereof, preferably according to SEQ ID No. 36 or 37, most preferably a cytoplasmic region of 4-1BB and mutants thereof.

In embodiments, the switchable CAR is a universal chimeric antigen receptor comprising a tag-binding domain.

In embodiments, the switchable CAR is a reversible chimeric antigen receptor comprising a tag. Advantageously, cells comprising a reversible universal chimeric antigen cell surface receptor are less exhausted after stimulation thereby improving persistence.

In further embodiments, the switchable chimeric antigen receptor comprises a further domain, wherein the further domain is a short peptide linker in the extracellular portion of the receptor that may serve to detect the chimeric antigen receptor on the cell surface or stimulate the chimeric antigen receptor T cell.

In preferred embodiments, the further domain forms a linear epitope for a monoclonal antibody (mab) specifically binding to the further domain. In some embodiments, the further domain comprises at least one linear epitope, preferably E7B6 according to SEQ ID No. 23 or SEQ ID No. 24.

In some embodiments, the further domain is located in between the tag-binding domain or the tag and the extracellular hinge domain or an integral part of the extracellular hinge domain.

Advantageously, the switchable CAR engrafted cells with the further domain can be specifically stimulated to proliferate preferentially and persist longer compared to non-engrafted cells either *in vitro* or *in vivo.* Further advantageously, the further domain may also be used to purify switchable CAR engrafted cells from mixed cell populations or to dampen switchable CAR engrafted cell-mediated immune response and to eliminate switchable CAR engrafted cells *in vivo.*

In further embodiments, the switchable CAR comprises a signal peptide. Advantageously, the signal peptide allows for expression on the cell surface of an effector cell. In embodiments, the signal peptide is located at the N-terminus of the switchable CAR nucleotide sequence in front of the tag-binding domain or the tag. In some embodiments, the signal peptide targets proteins to the secretory pathway either co-translationally or post-translationally and is selected from leader peptides from proteins like CD28, CD8α, IL-2, lysozyme C or the heavy or light chains of antibodies of human origin to avoid immunogenic reactions.

In embodiments, the tag-binding domain or tag is present at the amino-terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the switchable CAR comprises a sequence according to SEQ ID No. 39 to SEQ ID No. 56.

In embodiments, the switchable CAR is a reversible CAR and comprises a sequence according to SEQ ID No. 39 to SEQ ID No. 47, preferably according to SEQ ID No. 39, SEQ ID No. 40, SEQ ID No. 41, SEQ ID No. 44 or SEQ ID No. 46, more preferably according to SEQ ID No. 39, SEQ ID No. 40, SEQ ID No. 41 or SEQ ID No. 46.

In further embodiments, the switchable CAR is a UniCAR and comprises a sequence according to SEQ ID No. 48 to SEQ ID No. 56, preferably according to, SEQ ID No. 48, SEQ ID No. 49, SEQ ID No. 50 SEQ ID No. 53 or SEQ ID No. 55, more preferably according to SEQ ID No. 48, SEQ ID No. 49, SEQ ID No. 50 or SEQ ID No. 55.

According to the invention, after a period between 7 days and 21 days from the end of the administration period of the first dosage of the targeting module at least one further dosage of the targeting module is continuously administered to the subject with a dosage quantity in the range of 0.1 mg/day to 20 mg/day for a period between 4 days and 25 days.

In embodiments, the administering of the at least one further dosage of targeting module is carried out 7 days to 14 days after the administering of the first dosage of targeting module, preferably 7 days to 10 days after the administering of the first dosage of targeting module.

Preferably, after 7 days to 21 days after the first dosage of the targeting module at least one further dosage of the targeting module is continuously administered to the subject in the range of 0.1 mg/day to 20 mg/day to the subject for a period between 12 days and 20 days.

In preferred embodiments, the administering of the at least one further dosage of targeting module comprises the continuous administering of two to four further dosages of targeting module to the subject, each 7 days to 21 days after the preceding dosage, preferably each 7 days to 14 days after the preceding dosage.

In embodiments, the administering of the at least one further dosage of targeting module comprises three to four times continuous administering of targeting module each for 3 days to 4 days and in between each a break for 24 hours to 72 hours, preferably in between each a break for 24 hours to 48 hours.

In further embodiments, the kit for use in the treatment of hematological cancer according to the invention comprises at least one further targeting module,
wherein the at least one further targeting module comprises at least one hematological cancer cell-binding domain and a tag-binding domain or tag,
wherein the at least one hematological cancer cell-binding domain is an antibody, an antibody fragment, a protein, a peptide or a low molecular weight organic ligand that binds to surface antigens selected from the group comprising CD19, CD20, CD22, CD30, CD33, CD38, CD56, CD70, CD123, CD133, CD371, CXCR4, members of the mucin protein family and ligands of the NKG2D receptor,
wherein the targeting module and the at least one further targeting module comprise identical tag-binding domains or tags and different hematological cancer cell-binding domains, wherein the further targeting module is continuously administered to the subject 7 days to 21 days after the administering of the at least one further dosage of the targeting module with a dosage quantity in the range of 0.1 mg/day to 20 mg/day for a period between 4 days and 25 days.

A further aspect of the invention is a method for treating hematological cancer in a subject, preferably in a mammal, more preferably a human, by administration of a kit according to the invention, preferably to a subject in need thereof.

In embodiments, the method for treating hematological cancer in a subject comprises the following steps:
a. Continuous administering of a first dosage of a targeting module to a subject having hematological cancer with a dosage quantity in the range of 0.1 mg/day to 20 mg/day from day 0 of the treatment (beginning of treatment) for a period between 10 days and 25 days,
   wherein the targeting module comprises
   at least one hematological cancer cell-binding domain and
   a tag-binding domain or a tag,
b. Administering at least one dosage of a clinically effective amount of a T cell comprising a nucleotide sequence encoding a switchable CAR to the subject with a dosage quantity in the range of 1·10⁸ to 1·10⁹ cells once within day 0 to day 5 of the treatment,
   wherein the switchable CAR comprises
   a tag-binding domain or tag,
   an extracellular hinge and a transmembrane domain, and
   an intracellular signaling domain that comprises at least one signal transduction domain, and
c. 7 days to 21 days after the administering of the first dosage of the targeting module continuous administering of at least one further dosage of the targeting module to the subject with a dosage quantity in the range of 0.1 mg/day to 20 mg/day for a period between 4 days and 25 days,
   wherein the tag-binding domain of the targeting module binds to the tag of the switchable CAR or the tag of the targeting module binds to the tag-binding domain of the switchable CAR.

According to the invention, the method is carried out in the order of steps a., b. and c.

In embodiments, the targeting module and/or the T cell comprising a nucleotide sequence encoding a switchable CAR are administered in form of a pharmaceutical composition.

In embodiments, the hematological cancer is leukemia. In embodiments, the subject has acute myeloid leukemia (AML).

In embodiments, the subject is a mammal. In embodiments, the mammal is a human.

In embodiments, the peptide epitope tag is a myc-tag, a His-tag, a short linear peptide sequence from yeast transcription factor GCN4, preferably according to SEQ ID No. 18, SEQ ID No. 19 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 20 or SEQ ID No. 21 or mutants thereof; or a short linear peptide sequence from a human protein, preferably from a human nuclear protein, more preferably from the human La protein, even more preferably according to SEQ ID No. 22, SEQ ID No. 23, SEQ ID No. 24 or mutants thereof.

In embodiments, the tag-binding domain is an antibody, antigen-binding fragment, a protein or a peptide binding to a myc-tag, a His-tag, a short linear peptide sequence from yeast transcription factor GCN4, a leucine zipper sequence or a short linear peptide sequence from a human protein, preferably form a human nuclear protein, most preferably from the human La protein.

In embodiments, in step b. the clinically effective amount of the T cell comprising a nucleotide sequence encoding a switchable CAR is administrated with a dosage quantity in the range of 1·10⁸ to 5·10⁸ cells.

In embodiments, in step b. one dosage of the clinically effective amount of the T cell comprising a nucleotide sequence encoding a switchable CAR is administrated to the subject.

In embodiments, in step b. one dosage of a clinically effective amount of the T cell comprising a nucleotide sequence encoding a switchable CAR is administrated once on day 1 of the treatment.

In embodiments, in step b. one dosage of a clinically effective amount of the T cell comprising a nucleotide sequence encoding a switchable CAR is administrated 1 hour to 24 hours after starting the continuous administering of the targeting module in step a.

In embodiments, the hematological cancer cell-binding domain is an antibody, an antibody fragment, a protein, a peptide or a low molecular weight organic ligand that binds to surface antigens selected from the group comprising CD19, CD20, CD22, CD30, CD33, CD38, CD56, CD70, CD123, CD133, CD371, CXCR4, members of the mucin protein family and ligands of the NKG2D receptor.

In embodiments, the hematological cancer cell-binding domain is an antibody, an antibody fragment, a protein, a peptide or a low molecular weight organic ligand that binds to CD123.

In preferred embodiments, the CD123-binding domain is an antibody or antigen binding fragment.

In embodiments, the CD123-binding domain comprises the sequences SEQ ID No. 4 and SEQ ID No. 5.

In preferred embodiments, the CD123-binding domain comprises a sequence according to SEQ ID No. 6.

In embodiments, the hematological cancer cell-binding domain comprises a humanized amino acid sequence.

In embodiments, the targeting module has a length in the range of 20 to 1600 amino acids, preferably 200 to 800 amino acids.

In embodiments, the targeting module comprises one of the sequences according to SEQ ID No. 10 to SEQ ID No. 17.

In embodiments, the first dosage in step a. and/or the at least one further dosage of the targeting module in step c. is administered in the range of 0.5 mg/day to 8 mg/day, preferably in the range of 0.5 mg/day to 2 mg/day.

In embodiments, the first dosage of the targeting module in step a. is continuously administered for a period between 20 days and 24 days, preferably for a period of 20 days or 21 days.

In embodiments, the extracellular hinge and transmembrane domain comprise an extracellular hinge and a transmembrane domain of CD8α, CD28, ICOS (CD278) or mutants and combinations thereof.

In embodiments, the intracellular signaling domain comprises at least one signal transduction domain selected from the group consisting of a cytoplasmic region of CD3, CD28, 4-1BB (CD137), ICOS (CD278), IL-2, IL-7, IL-15 or IL-21 and mutants thereof.

In embodiments, the method further comprises prior to the step a. a lymphodepletion. As used herein, the term "lymphodepletion" means a chemotherapy to reduce the lymphocytes in the blood and bone marrow of the subject. Preferably, lymphodepletion is carried out directly on the days before CAR-T cell administration according to step b.

According to the method according to the invention, the step c. is carried out 7 days to 21 days after step a., preferably 7 days to 14 days, more preferably 7 days to 10 days.

In embodiments, step c. comprises the continuous administering of at least one further dosage of the targeting module for a period between 12 days and 20 days.

In embodiments, step c. comprises the continuous administering of two to four further dosages of targeting module to the subject, each after 7 days to 21 days, preferably 7 days to 14 days.

In embodiments, step c. comprises the continuous administering of three to four times continuous administering of targeting module each for 3 days to 4 days and in between each a break for 24 hours to 72 hours, preferably in between each a break for 24 hours to 48 hours.

In embodiments, the targeting module in step a. and/or step c. is continuously administered until a stable plasma concentration of targeting module is reached.

In embodiments, the targeting module in step a. and/or step c. is continuously administered until side effects occur.

In embodiments, the method according to the invention is carried out in combination with a therapy selected from the group comprising chemotherapy, radiation, immunosuppressive agents, antibodies and other immunoablative agents.

In further embodiments, the method further comprises the administering of at least one further targeting module, wherein the at least one further targeting module comprises at least one hematological cancer cell-binding domain and a tag-binding domain or tag,
wherein the at least one hematological cancer cell-binding domain is an antibody, an antibody fragment, a protein, a peptide or a low molecular weight organic ligand that binds to surface antigens selected from the group comprising CD19, CD20, CD22, CD30, CD33, CD38, CD56, CD70, CD123, CD133, CD371, CXCR4, members of the mucin protein family and ligands of the NKG2D receptor,
wherein the targeting module and the at least one further targeting module comprise identical tag-binding domains or tags and different hematological cancer cell-binding domains,
wherein the further targeting module is continuously administered to the subject 7 days to 21 days after the administering of the at least one further dosage of the targeting module with a dosage quantity in the range of 0.1 mg/day to 20 mg/day for a period between 4 days and 25 days.

Another aspect of the invention is a nucleic acid, vector or cell comprising a nucleotide sequence encoding the switchable CAR for use in a method for treating hematological cancer in a subject and/or for use in a kit for use in the treatment of hematological cancer according to the invention.

As used herein, "nucleic acid" or "nucleic acid molecule" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Nucleic acids can be either single stranded or double stranded. Nucleotides are designated as follows: one-letter code is used for designating the base of a nucleoside: a is adenine, t is thymine, c is cytosine, and g is guanine.

In embodiments, the nucleic acid, vector and/or cell are isolated. In embodiments, the nucleic acid is a cDNA. As used herein, the term "cDNA" (complementary DNA) refers to doublestranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

In embodiments, the vector is selected from the group comprising a DNA vector, an RNA vector, a plasmid, a lentiviral vector, retroviral vector, adenoviral vector and an adeno-associated viral vector.

In embodiments, the vector further comprises a promoter, wherein the promoter is selected from the group comprising an EF-1 promoter, a CMV IE gene promoter, an EF-1α promoter, an ubiquitin C promoter, or a phosphoglycerate kinase (PGK) promoter.

In embodiments, the cell comprises an exogenous nucleotide sequence encoding the switchable CAR that is expressed on the surface of the cell.

In embodiments, the cell is an immune effector cell selected from the group comprising a T cell, preferably a cytotoxic T lymphocyte (CTL) or regulatory T cell (T_{reg}); Natural Killer (NK) cell, and macrophage (MP).

The invention is not limited to the embodiments shown and described, but also includes all embodiments having the same effect within the meaning of the invention. Furthermore, the invention is also not limited to the specifically described combinations of features, but may also be defined by any other combination of specific features of all the individual features disclosed as a whole, provided that the individual features are not mutually exclusive, or a specific combination of individual features is not explicitly excluded.

### Figures and Examples

The present invention will now be further explained by the following non-limiting figures and examples.
Fig. 1 shows a schematic representation of a design of the phase 1A study using the kit for use in the treatment of hematological cancer comprising three consolidation cycles (CC) after the initiation cycle (IC) of targeting module administration.
**Fig. 2** shows exemplary measurement of A) leukocytes (white blood cells, WBC) and B) neutrophils over the course of treatment.
**Fig. 3** shows the UniCAR-T-cell numbers in peripheral blood (PB) and bone marrow (BM) over the course of treatment comprising the administering of targeting module (core cycle) and one further administering of targeting module (add. cycle). Cell number was calculated from vector copy number determined by digital droplet polymerase chain reaction, mean and standard deviation are shown.
**Fig. 4** shows a comparison of UniCAR-T expansion and achieved responses in patients receiving one further administration of targeting module (add. cycle) after a shorter or longer break.
**Fig. 5** shows testing of encephalopathy (CRES) using a writing test.
**Fig. 6** shows transaminase (ASAT and ALAT) and ferritin values during treatment and after stop of targeting module infusion.

### Switchable CAR T cells

Immune cells can be genetically engineered to express switchable CARs. For the genetical engineering to express UniCARs or RevCARs, a polynucleotide vector encoding the UniCAR or RevCAR and all necessary elements to ensure its expression in the genetically engineered immune cell is transferred into the immune cell. In particular, the UniCAR and RevCAR comprises IL-2LP (modified human IL-2 leader peptide), a tag-binding domain or tag, G₄S₁ (glycine-serine linker), ECD (extracellular domain), TMD (transmembrane domain) and at least two ICDs (intracellular domains) according to SEQ ID No. 30 to SEQ ID No. 38.

The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and co-transfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag containing molecules for surface expression of switchable CARs or mabs directed against a further domain of switchable CARs from day 3 onwards after the final administration of virus supernatant. Switchable CAR transduced T cells can be propagated *in vitro* by culturing them under the supply of recombinant cytokines and activating anti-CD3 mabs.

In case the switchable CAR harbors the optional further domain, a peptide sequence forming a linear epitope for a mab, immune cells genetically modified to express switchable CARs can be specifically propagated *in vitro* by coating a mab or antibody fragments thereof binding to the further domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR-carrying immune cells and therefore enrichment of switchable CAR genetically modified immune cells in a mixed population.

The further domain, a peptide sequence forming a linear epitope for a mab, can be further utilized to enrich and purify switchable CAR-expressing immune cells from mixed populations. Enrichment and purification are performed with the help of a mab or antibody fragment thereof binding to the further switchable CAR domain to either mark switchable CAR-expressing cells for cell sorting or to transiently link the switchable CAR expressing immune cell to small particles, which can be utilized for cell isolation. In one aspect, switchable CAR-engrafted immune cells are incubated with the mab recognizing the further domain. Next, magnetic beads are added, which are conjugated with antibodies or fragments thereof directed against the species- and isotype-specific heavy and light chains of the mab binding to the further domain. Thus, switchable CAR-expressing immune cells and magnetic beads are linked and are trapped and separated from other immune cells in a magnetic field.

### Targeting module

The targeting module TM123 is a soluble, recombinant fusion protein comprising two antibody-derived binding domains. One selectively binds to the target antigen CD123, the other recognizes the tag-binding domain or tag presented on the switchable CAR expressing cells (epitope E5B9 from the human La protein). Thus, TM123 functions as a bridging module between switchable CAR-T and a CD123-expressing target cancer cell. The targeting module further comprises an 8x-histidine tag for detection and purification purposes at the C-terminus.

### In vitro characterization

Successful manufacturing of switchable CARs can be proven by immunostaining of CARs using flow cytometry and EGFP expression.

### Cytotoxicity assay

The potency of the switchable CARs and CD123-binding TMs to induce a tumor cell elimination can be tested using a suspension cell-based co-cultivation assay, e.g. with Nalm-6 target cells or BxPC3 target cells in the presence of targeting module. Target cells can be quantified by flow cytometry or lncuyte^{®}Zoom. For flow cytometry-based cytotoxicity lysis is calculated normalizing the cell count of each sample to a control sample where only tumor cells were plated. For Incucyte^{®} -based cytotoxicity both living and dead target cells are quantified and lysis is assessed by calculating the proportion of dead target cells in relation to total target cells.

### In vivo characterization

The switchable CARs can be characterized *in vivo* using mice, wherein 1·10⁵ MV4-11 luc cells are injected intravenously. Three days later, 5·10⁶ RevCAR T cells are injected intravenously and targeting module administration starts, e.g. with anti-CD123 targeting module administered intraperitoneally (IP) twice a day (1 µg/g or 3 µg/g mouse) for a total of three weeks on weekdays. Tumor progression can be checked by fluorescence imaging using the IVISO SpectrumCT system. Mice have to be checked daily for signs of disease progression. If mice reach endpoint criteria, mice are sacrificed, and organs analyzed by flow cytometry.

### In vivo characterization in humans - Design of the phase 1A study

UniCAR-T cells are manufactured from autologous starting material (LEU), patients are allowed to receive bridging therapy if required; lymphodepletion (LD) with standard dose Flu/Cy is performed prior to start of treatment; TM123 is administered as continuous intravenous infusion over 21 or 24 days starting at day 0 followed by UniCAR-T administration at day 1 (core cycle). Patients were allowed to receive a 2nd cycle (further cycle) of TM123 based upon initial safety and efficacy read-out in the core cycle.

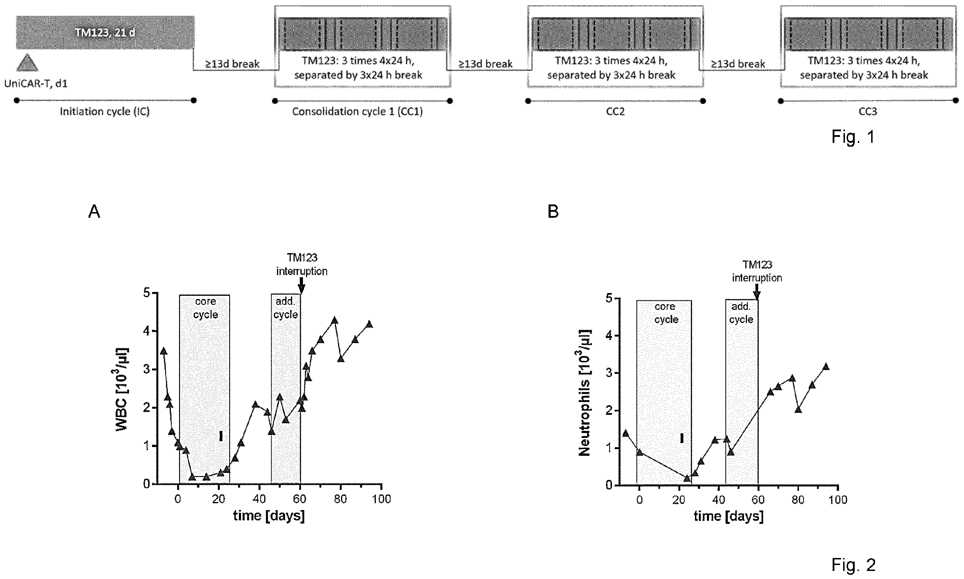

Fig. 2 shows the rapid recovery of leukocytes (WBC) and neutrophils after the end of targeting module administration.

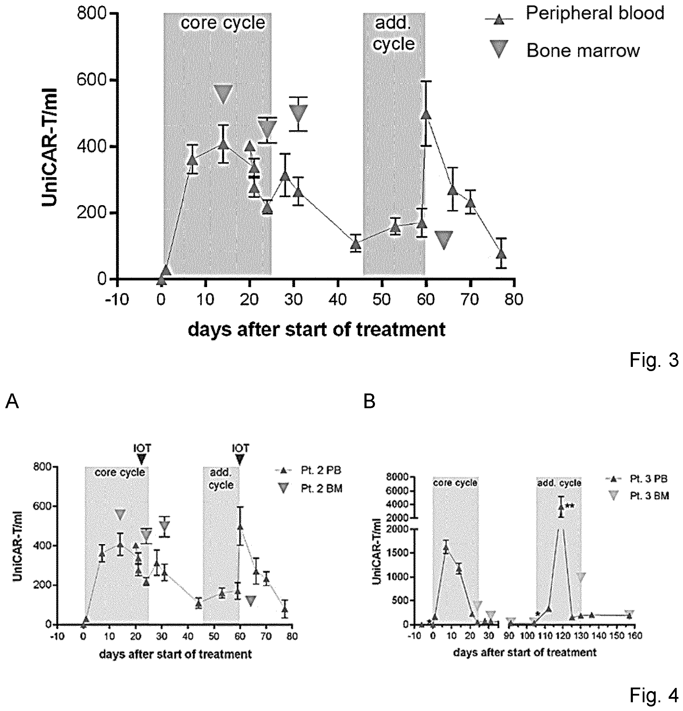

Fig. 3 shows the UniCAR-T-cell numbers in peripheral blood (PB) and bone marrow (BM) over the course of treatment comprising the administering of targeting module (core cycle) and one further administering of targeting module (add. cycle). Cell number was calculated from vector copy number determined by digital droplet polymerase chain reaction, mean and standard deviation are shown. Fig. 3 demonstrates the re-expansion of UniCAR-T cells during an additional TM123 cycle.

**Tab. 1 shows results of UniCAR-T expansion and achieved responses in patients receiving one further administration of targeting module (add. cycle) after a shorter (patient 2: 21 d) or longer break (patient 3: 81 d).**

| | % bone marrow blasts pre-treatment | % bone marrow blasts after core cycle / response | % bone marrow blasts after add. Cycle / response |
|---|---|---|---|
| Patient (Pt.) 2 | 20 | 0 | < 5 |
| Patient (Pt.) 3 | 30 | 2 | 53 |

This comparison shows improved results for using breaks between the core cycle and the additional cycle according to the invention.

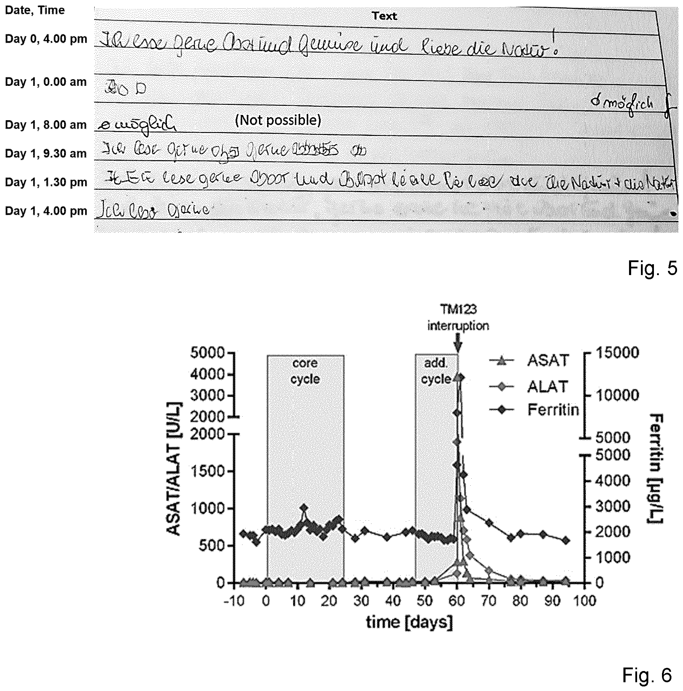

Fig. 5 shows testing of encephalopathy (CRES) resulted from treatment using a writing test. Fig. 5 shows the improvement of writing ability after the ending of the targeting module cycle and therefore proves the effectivity of the switch-off mechanism of the treatment.

### Cited non-patent literature

Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Frigault MJ, Lee J, Basil MC, Carpenito C, Motohashi S, Scholler J, Kawalekar OU, Guedan S, McGettigan SE, Posey AD Jr, Ang S, Cooper LJ, Platt JM, Johnson FB, Paulos CM, Zhao Y, Kalos M, Milone MC, June CH (2015) Identification of chimeric antigen receptors that mediate constitutive or inducible proliferation of T cells. Cancer Immunol. Res. 3 (4), 356-367.
Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH (2009) Chimeric receptors containing CD137 signal transduction domains mediate enhanced survival of T cells and increased antileukemic efficacy in vivo. Mol. Ther. 17 (8), 1453-1464.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Pinthus JH, Waks T, Kaufman-Francis K, Schindler DG, Harmelin A, Kanety H, Ramon J, Eshhar Z (2003) Immuno-gene therapy of established prostate tumors using chimeric receptor-redirected human lymphocytes. Cancer Res. 63 (10), 2470-2476.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Wang J, Jensen M, Lin Y, Sui X, Chen E, Lindgren CG, Till B, Raubitschek A, Forman SJ, Qian X, James S, Greenberg P, Riddell S, Press OW (2007) Optimizing adoptive polyclonal T cell immunotherapy of lymphomas, using a chimeric T cell receptor possessing CD28 and CD137 costimulatory domains. Hum. Gene Ther. 18 (8), 712-725.
Weber EW, Parker KR, Sotillo E, Lynn RC, Anbunathan H, Lattin J, Good Z, Belk JA, Daniel B, Klysz D, Malipatlolla M, Xu P, Bashti M, Heitzeneder S, Labanieh L, Vandris P, Majzner RG, Qi Y, Sandor K, Chen LC, Prabhu S, Gentles AJ, Wandless TJ, Satpathy AT, Chang HY, Mackall CL (2021) Transient rest restores functionality in exhausted CAR-T cells through epigenetic remodeling. Science 372 (6537), eaba1786.
Zhang T, Lemoi BA, Sentman CL (2005) Chimeric NK-receptor-bearing T cells mediate antitumor immunotherapy. Blood. 106 (5), 1544-1551.
Zhao Y, Wang QJ, Yang S, Kochenderfer JN, Zheng Z, Zhong X, Sadelain M, Eshhar Z, Rosenberg SA, Morgan RA (2009) A herceptin-based chimeric antigen receptor with modified signaling domains leads to enhanced survival of transduced T lymphocytes and antitumor activity. J. Immunol. 183 (9), 5563-5574.

## Claims

1. A kit for use in the treatment of hematological cancer comprising:
a. a T cell comprising a nucleotide sequence encoding a switchable chimeric antigen receptor (CAR), wherein the switchable CAR comprises
a tag-binding domain or tag,
an extracellular hinge and a transmembrane domain, and
an intracellular signaling domain that comprises at least one signal transduction domain; and
b. a targeting module comprising
at least one hematological cancer cell-binding domain and
a tag-binding domain or a tag,
wherein the tag-binding domain of the targeting module binds to the tag of the switchable CAR or the tag of the targeting module binds to the tag-binding domain of the switchable CAR,
wherein a first dosage of the targeting module is continuously administered to a subject having hematological cancer with a dosage quantity in the range of 0.1 mg/day to 20 mg/day from day 0 of the treatment for a period between 10 days and 25 days, and
wherein at least one dosage of a clinically effective amount of the T cell comprising a nucleotide sequence encoding a switchable CAR is administered to the subject with a dosage quantity in the range of 1·10⁸ to 1·10⁹ cells within day 0 to day 5 of the treatment, and
wherein 7 days to 21 days after the administering of the first dosage of the targeting module at least one further dosage of the targeting module is continuously administered to the subject with a dosage quantity in the range of 0.1 mg/day to 20 mg/day for a period between 4 days and 25 days.

2. The kit for use in the treatment of hematological cancer of claim 1, wherein the targeting module and/or the T cell comprising a nucleotide sequence encoding a switchable CAR are administered in form of a pharmaceutical composition.

3. The kit for use in the treatment of hematological cancer of claim 1 or 2, wherein the subject has acute myeloid leukemia (AML).

4. The kit for use in the treatment of hematological cancer of one of the claims 1 to 3, wherein the tag is a myc-tag, a His-tag, a short linear peptide sequence from yeast transcription factor GCN4, a leucine zipper sequence or a short linear peptide sequence from a human protein.

5. The kit for use in the treatment of hematological cancer of one of the claims 1 to 4, wherein the tag is a short linear peptide sequence from the human La protein.

6. The kit for use in the treatment of hematological cancer of one of the claims 1 to 5, wherein the tag-binding domain is an antibody, antigen-binding fragment, a protein or a peptide binding to a myc-tag, a His-tag, a short linear peptide sequence from yeast transcription factor GCN4, a leucine zipper sequence or a short linear peptide sequence from a human protein.

7. The kit for use in the treatment of hematological cancer of one of the claims 1 to 6, wherein the at least one dosage of a clinically effective amount of the T cell comprising a nucleotide sequence encoding a switchable CAR is administrated with a dosage quantity in the range of 1·10⁸ to 5·10⁸ cells.

8. The kit for use in the treatment of hematological cancer of one of the claims 1 to 7, wherein one dosage of a clinically effective amount of a T cell comprising a nucleotide sequence encoding a switchable CAR is administrated to the subject.

9. The kit for use in the treatment of hematological cancer of claim 8, wherein one dosage of a clinically effective amount of the T cell comprising a nucleotide sequence encoding a switchable CAR is administrated once on day 1 of the treatment.

10. The kit for use in the treatment of hematological cancer of one claim 8 or 9, wherein one dosage of a clinically effective amount of the T cell comprising a nucleotide sequence encoding a switchable CAR is administrated 1 hour to 24 hours after starting the continuous administering of the targeting module.

11. The kit for use in the treatment of hematological cancer of one of the claims 1 to 10, wherein the hematological cancer cell-binding domain is an antibody, an antibody fragment, a protein, a peptide or a low molecular weight organic ligand that binds to surface antigens selected from the group comprising CD19, CD20, CD22, CD30, CD33, CD38, CD56, CD70, CD123, CD133, CD371, CXCR4, members of the mucin protein family and ligands of the NKG2D receptor.

12. The kit for use in the treatment of hematological cancer of one of the claims 1 to 11, wherein the hematological cancer cell-binding domain is an antibody, an antibody fragment, a protein, a peptide or a low molecular weight organic ligand that binds to CD123.

13. The kit for use in the treatment of hematological cancer of one of the claims 1 to 12, wherein the hematological cancer cell-binding domain comprises a humanized amino acid sequence.

14. The kit for use in the treatment of hematological cancer of one of the claims 1 to 13, wherein the targeting module has a length in the range of 20 to 1600 amino acids.

15. The kit for use in the treatment of hematological cancer of one of the claims 1 to 14, wherein the first dosage and/or the at least one further dosage of the targeting module is administered with a dosage quantity in the range of 0.5 mg/day to 8 mg/day.

16. The kit for use in the treatment of hematological cancer of one of the claims 1 to 15, wherein the first dosage of the targeting module is continuously administered for a period between 20 days and 24 days.

17. The kit for use in the treatment of hematological cancer of one of the claims 1 to 16, wherein the extracellular hinge and transmembrane domain comprise an extracellular hinge and a transmembrane domain of CD8α, CD28, ICOS (CD278) or mutants and combinations thereof.

18. The kit for use in the treatment of hematological cancer of one of the claims 1 to 17, wherein the intracellular signaling domain comprises at least one signal transduction domain selected from the group consisting of a cytoplasmic region of CD3, CD28, 4-1BB (CD137), ICOS (CD278), IL-2, IL-7, IL-15 or IL-21 and mutants thereof.

19. The kit for use in the treatment of hematological cancer of one of the claims 1 to 18, wherein the administering of the at least one further dosage of targeting module is carried out 7 days to 14 days after the administering of the first dosage of targeting module.

20. The kit for use in the treatment of hematological cancer of one of the claims 1 to 19, wherein the administering of the at least one further dosage of targeting module comprises the continuous administering of two to four further dosages of targeting module to the subject, each 7 days to 14 days after the administering of the preceding dosage.

21. The kit for use in the treatment of hematological cancer of one of the claims 1 to 20, wherein the administering of the at least one further dosage of targeting module comprises three to four times continuous administering of targeting module each for 3 days to 4 days and in between each a break of 24 hours to 72 hours.
